# EUROPÄISCHE PATENTSCHRIFT

(11) **EP 3 223 868 B1**
(45) Veröffentlichungstag und Bekanntmachung des Hinweises auf die Patenterteilung: **13.11.2019**
(21) Anmeldenummer: 15801823.4
(22) Anmeldetag: 27.11.2015
(51) Int. Cl.: A61L 2/26

(54) **VERSCHLUSS FÜR EINEN MEDIZINISCHEN STERILBEHÄLTER SOWIE STERILBEHÄLTER MIT EINEM DERARTIGEN VERSCHLUSS**
CLOSURE ELEMENT FOR A MEDICAL STERILE CONTAINER, AND STERILE CONTAINER WITH SUCH A CLOSURE ELEMENT
FERMETURE POUR CONTENANT MÉDICAL STÉRILE ET CONTENANT STÉRILE COMPRENANT UNE FERMETURE DE CE TYPE

(30) Priorität: 28.11.2014 DE 102014117516
(43) Veröffentlichungstag der Anmeldung: 04.10.2017
(73) Patentinhaber: Aesculap AG, 78532 Tuttlingen (DE)
(72) Erfinder: THOMAS, Stefan, 78532 Tuttlingen (DE); BELLIKLI, Serkan, 72355 Schömberg (DE); SCHWEIZER, Matthias, 78532 Tuttlingen (DE); SCHUSTER, Stefan, 79865 Grafenhausen (DE); WEISSHAUPT, Dieter, 78194 Immendingen (DE)
(74) Vertreter: Winter, Brandl, Fürniss, Hübner, Röss, Kaiser, Polte - Partnerschaft mbB
(86) Internationale Anmeldenummer: PCT/EP2015/077956
(87) Internationale Veröffentlichungsnummer: WO 2016/083595

(56) Entgegenhaltungen:
- EP-A1- 1 857 375
- WO-A1-92/01480
- WO-A1-2013/135658
- WO-A1-2014/029587
- DE-A1- 4 411 970
- DE-A1-102004 028 040
- DE-U1- 9 203 630
- DE-U1- 29 907 481
- DE-U1-202012 002 487
- US-A- 4 915 913

## Beschreibung

Die vorliegende Erfindung betrifft einen Verschluss für einen medizinischen Sterilbehälter mit einem wannenartigen ersten Behälterteil und einem deckelartigen zweiten Behälterteil mit einer schwenkbar zwischen einer Schließstellung, in der die Behälterteile mittels des Verschlusses miteinander verriegelt sind, und einer Offenstellung, in der die Behälterteile unverriegelt sind, an einem der Behälterteile anordbaren Verschlussklappe sowie einen medizinischen Sterilbehälter mit einem derartigen Verschluss.

Sterilbehälter mit einem solchen Verschluss werden in der Medizin, insbesondere in der Chirurgie, genutzt, um chirurgische Instrumente, Implantate und dergleichen zu sterilisieren und nach der Sterilisation kurzzeitig zu lagern und zu transportieren. Zu sterilisierende Gegenstände werden in dem Behälterinnenraum angeordnet. Der Behälter wird durch Anordnen des Behälterdeckels an dem Behälterunterteil verschlossen und mit den darin aufgenommenen zu sterilisierenden Gegenständen einem Sterilisator zugeführt, in dem der Behälterinnenraum mit einem sterilisierenden Gas, z.B. Wasserdampf, beaufschlagt wird. Um Rekontamination nach erfolgter Sterilisierung vermeiden und Sterilität der in dem Behälter aufgenommenen Gegenstände sicherstellen zu können, ist es bekannt, Behälter nach der Sterilisierung zu verplomben.

Aus der DE 92 03 630 U1 ist ein Spannverschluss für chirurgische Sterilisationsbehälter und andere Behältnisse bekannt, der eine Verschlusslasche hat, welche von einer Offen- in eine Schließstellung und umgekehrt verschwenkbar ist und in ihrer Schließstellung ein vorzugsweise wannenförmiges erstes Behälterteil mit einem als Deckel ausgebildeten zweiten Behälterteil verbindet, wobei der Spannverschluss als Kniehebel- oder Exzenterverschluss ausgebildet ist, dessen Verschlusslasche dazu ein an dem einen Behälterteil verschwenkbar gelagertes Spannlaschenteil hat, an dem mit Abstand zur Schwenkachse ein Zuglaschenteil schwenkbar gehalten ist, welches in Schließstellung des Spannverschlusses einen am anderen Behälterteil vorgesehenen Schließhaken hintergreift, wobei das Zuglaschenteil auf seiner zum Schließhaken weisenden Seite einen in Schließstellung den Schließhaken über- oder hintergreifenden innenseitigen Laschenvorsprung oder dergleichen nach innen abgewinkelten Randbereich hat.

Aus der DE 31 16 036 C2 ist ein Sterilisationsbehälter mit einem Deckel, der durch einen Verschluss geschlossen gehalten werden kann, bekannt, wobei der Verschluss einen Hebel mit einem Schlitz aufweist, der zum Verschließen auf einer am Behälter oder dem Deckel befestigte Öse derart aufsetzbar ist, dass die Öse nach dem Verschließen von dem Hebel vorsteht und durch sie hindurch ein Indikatorklebstreifen hindurchführbar ist.

Aus der DE 90 07 070 U1 ist eine Plombe zum Sichern eines Verschlusses eines Sterilisationsbehälters bekannt, die aus einem Schild und einem mit zwei Enden unlösbar mit dem Schild verbindbaren Verbindungselement besteht.

Die DE 20 2012 002 487 U1 offenbart einen Verschluss gemäß dem Oberbegriff des Anspruchs 1. Weiterer für die vorliegende Erfindung relevanter Stand der Technik findet sich in den Druckschriften DE 10 2004 028040 A1, DE 92 03 630 U1, WO 2013/135658 A1, WO 2014/029587 A1, US 4 915 913 A, DE 44 11 970 A1, EP 1 857 375 A1, WO 92/01480 A1, DE 299 07 481 U1 und DE 197 55 532 A1.

Bei bekannten Verschlüssen und Sterilisationsbehältern ist nachteilig, dass eine Plombe ohne Öffnen des Verschlusses entfernt und unter Umständen durch eine neue Plombe ersetzt werden kann. Sterilität ist daher auch bei einem verplombten Behälter nicht sichergestellt. Des Weiteren kann gegebenenfalls bei einer üblichen Handhabung eines Sterilisationsbehälters ein Verschluss versehentlich geöffnet oder eine Plombe versehentlich entfernt werden. Bei einem verschlossenen Behälter ohne Plombe kann daher nicht nachvollzogen werden, ob der Behälter bereits geöffnet wurde und die Sterilität fraglich ist, oder ob lediglich die Plombe entfernt, der Behälter jedoch nicht geöffnet wurde. Ein anderer Nachteil ist, dass ein Anwender beim Öffnen eines verplombten Behälters zwei Handhabungsprozesse durchzuführen hat, einmal das Entfernen der Plombe und einmal das Öffnen des Verschlusses.

Ausgehend von dem vorstehend beschriebenen Stand der Technik liegt der Erfindung die Aufgabe zugrunde, einen Verschluss für einen medizinischen Sterilbehälter sowie einen Sterilbehälter bereitzustellen, der sicher zu verschließen ist, eine verlässliche Sicherung gegen ein unbeabsichtigtes oder unbefugtes Öffnen besitzt und Rückschlüsse darauf ermöglicht, ob der Behälter geöffnet wurde oder nicht, so dass eine Sterilität des Sterilbehälters und der darin aufgenommenen Objekte bis zum Öffnen des Sterilbehälters gewährleistet ist.

Diese Aufgabe wird nach der vorliegenden Erfindung gelöst durch einen Verschluss für einen medizinischen Sterilbehälter mit den Merkmalen des Anspruchs 1 und einen medizinischen Sterilbehälter mit den Merkmalen des Anspruchs 10.

Diese Aufgabe wird nach der vorliegenden Erfindung somit zum einen gelöst durch einen Verschluss für einen medizinischen Sterilbehälter mit einem wannenartigen ersten Behälterteil und einem deckelartigen zweiten Behälterteil, insbesondere für einen Sterilbehälter für medizinische Instrumente und dergleichen mit einem wannenförmigen Behälterunterteil mit einem Behälterboden und einer Behälterwand und einem Behälterdeckel zum Anordnen an der Behälterwand und zum Verschließen des Behälterunterteils, mit einer schwenkbar zwischen einer Schließstellung, in der die Behälterteile mittels des Verschlusses miteinander verriegelt sind, und einer Offenstellung, in der die Behälterteile unverriegelt sind, an einem der Behälterteile anordbaren Verschlussklappe, wobei der Verschluss ein Riegelelement aufweist, das die Verschlussklappe in der Schließstellung des Verschlusses mit dem anderen Behälterteil verriegelt. Durch das den Verschluss in der Schließstellung verriegelnde Riegelelement ist ein versehentliches Öffnen des Verschlusses nicht möglich.

Das Riegelelement ist erfindungsgemäß verrückbar, insbesondere linear verrückbar oder rotatorisch verrückbar, an der Verschlussklappe angeordnet. Das Riegelelement ist zwischen seiner den Verschluss verriegelnden Stellung und einer den Verschluss freigebenden Stellung verrückbar. Das Riegelelement kann vorzugsweise relativ zur Verschlussklappe zwischen einer Riegelposition, in der das Riegelelement mit dem anderen Behälterteil, insbesondere mit dem Behälterunterteil, vorzugsweise dessen Behälterwand oder einer daran angeordneten Trägerplatte, verriegelt ist, und einer Freigabeposition, in der das Riegelelement von dem anderen Behälterteil entriegelt ist, positionierbar sein. Zum Öffnen des Verschlusses ist eine bewusste Betätigung des Riegelelements durch einen Nutzer erforderlich, damit das Riegelelement und damit der Verschluss entriegelt werden und der Verschluss geöffnet werden kann.

In einer vorteilhaften Ausführungsform der Erfindung ist das Riegelelement rotatorisch verrückbar an der Verschlussklappe angeordnet. Insbesondere kann das Riegelelement U-förmig ausgebildet sein und an seinen jeweiligen Enden Riegelabschnitte aufweisen, die in seitlichem Eingriff mit einem Eingriffsabschnitt des anderen Behälterteils bringbar sind. Ferner kann ein Verbindungsabschnitt der beiden Riegelabschnitte elastisch ausgebildet sein und jeder Riegelabschnitt drehbar an der Verschlussklappe angebunden sein. Dies hat den Vorteil, dass ein beidseitiger Eingriff des Riegelelements in die Verschlussklappe erfolgen kann. Die Sicherheit gegen ein unbeabsichtigtes Öffnen kann somit erhöht werden.

Bei einer Ausführungsform kann das Riegelelement in die Riegelposition vorgespannt sein, insbesondere mittels einer Feder. Aufgrund der Vorspannung kann das Riegelelement nicht versehentlich entriegelt werden. Eine Betätigung zum Entriegeln ist bewusst gegen die Vorspannungskraft durchzuführen.

Bei einer Ausführungsform kann das Riegelelement eine Sperrklinke oder Raststruktur aufweisen, die in der Schließstellung des Verschlusses mit dem anderen Behälterteil verrastet ist, insbesondere bei in der Riegelposition befindlichem Riegelelement mit dem anderen Behälterteil verrastet ist. Unter einer Sperrklinke ist ein Element oder eine Einheit zu verstehen, die eine dazu passend geformte Gegenstruktur am anderen Behälterteil hintergreift oder darin eingreift und eine Bewegung des Verschlusses aus der Schließstellung heraus verhindert. Mittels der Sperrklinke ist der Verschluss in der Schließstellung gesichert.

Es ist besonders vorteilhaft, wenn die Verschlussklappe eine Ausnehmung aufweist und das Riegelelement eine Ausnehmung aufweist, wobei bei in der Riegelposition befindlichem Riegelelement eine Plombe, insbesondere eine Plombe wie sie in der DE 10 2012 004 961 A1 beschrieben ist, zum Verplomben des medizinischen Sterilbehälters in den Ausnehmungen anordbar ist. Es ist insbesondere vorteilhaft, wenn die Plombe im montierten Zustand in der Ausnehmung des Riegelelements versenkt ist und bündig dazu abschließt. Alternativ oder zusätzlich kann eine Plombe nur am Sterilbehälter und in den Ausnehmungen angeordnet und befestigt werden, wenn sich der Verschluss in der Schließstellung befindet, also bei mittels des Verschlusses verschlossenem Sterilbehälter. Es kann insbesondere möglich sein, eine Plombe nicht am Sterilbehälter und in den Ausnehmungen befestigen und anordnen zu können, wenn der Verschluss nicht korrekt verschlossen ist, insbesondere nicht, wenn sich das Riegelelement nicht in der Riegelposition befindet. Anders ausgedrückt kann der Sterilbehälter nur verplombt werden, wenn der Verschluss korrekt geschlossen ist und sich das Riegelelement in der Riegelposition befindet. Ist der Sterilbehälter erst einmal verplombt worden, ist die dazu verwendete Plombe derart in den Ausnehmungen aufgenommen, dass sie nicht für eine Handhabung, insbesondere für ein Öffnen, zugänglich ist. Vorzugsweise kann die Plombe nur durch eine nutzerseitige Betätigung des Riegelelements geöffnet oder entfernt werden, nämlich durch ein nutzerseitiges Verrücken des Riegelelements aus der Riegelposition in die Freigabeposition. Anders ausgedrückt wird die Plombe beim bzw. durch das Öffnen des Verschlusses zwangsläufig zerstört. Es ist damit zum einen sichergestellt, dass ein mit einer unzerstörten Plombe versehener Sterilbehälter mit 100-prozentiger Sicherheit nach dem Verplomben nicht bereits geöffnet wurde. Zum anderen ist sichergestellt, dass ein Sterilbehälter, dessen Plombe zerstört ist, mit großer Wahrscheinlichkeit geöffnet und mit 100-prozentiger Sicherheit zumindest entriegelt war.

Es ist des Weiteren von Vorteil, wenn das Riegelelement, insbesondere die Sperrklinke, eine Anlaufschräge aufweist, über die das Riegelelement beim Verschließen des Behälters aus der Riegelposition verschiebbar ist. Insbesondere im Falle eines in seine Riegelposition vorgespannten Riegelelements ist so ein Verschließen des Verschlusses besonders einfach, nämlich indem der Verschluss einfach in seine Schließstellung überführt wird. Sobald die Anlaufschräge die zum Verriegeln vorgesehene Gegenstruktur am Behälterteil erreicht, wird das Riegelelement bei fortgesetzter Schließbewegung gegen die Vorspannung aus seiner Riegelposition in Richtung der Freigabeposition gedrängt, bis es die Riegelposition erreicht hat. Spätestens dann ist die Anlaufschräge freigegeben und das Riegelelement schnappt aufgrund seiner Vorspannung in die Riegelposition zurück und verriegelt den Verschluss in der Schließstellung.

Erfindungsgemäß ist an der Verschlussklappe eine Schließlasche kniehebelartig schwenkbar angeordnet. Die Schließlasche ist ausgebildet, um in der Schließstellung des Verschlusses mit dem anderen Behälterteil in Eingriff zu stehen. Ein solcher Verschluss ermöglicht in vorteilhafter Weise bei relativ geringen Betätigungskräften relativ hohe Schließkräfte, so dass ein dichtes Verschließen des Behälters, insbesondere wenn dieser zwischen Behälterunterteil und Deckel eine elastische Dichtung besitzt, in einfacher Weise realisiert werden kann. Die Schließlasche kann in der Schließstellung des Verschlusses an der Verschlussklappe anliegen. Auf diese Weise ist auch ein bündiges Anliegen des Verschlusses am Behälter möglich, was für Transport und Lagerung des Behälters von Vorteil ist. Die Schließklappe kann in einer Offenstellung des Verschlusses von der Verschlussklappe abgeschwenkt sein. So ist ein Eingreifen der Schließlasche in eine dazu vorgesehene Gegenstruktur am Behälterteil und ein Verbringen des Verschlusses in die Schließstellung besonders einfach möglich.

Das Riegelelement kann in besonders vorteilhafter Weise die Verschlussklappe und die Schließlasche in der Schließstellung des Verschlusses zumindest abschnittsweise umgreifen und zueinander lagefixieren. Dadurch ist ein Bewegen des Verschlusses ohne vorheriges Verbringen des Riegelelements aus seiner Riegelposition in die Freigabeposition nicht möglich, was zur vorgenannten Verriegelung des Riegelelements mit dem entsprechenden Behälterteil eine zusätzliche Sicherung und damit Sicherheit bedeutet.

Bei einer weiteren Ausführungsform ist die Schließlasche in eine von der Verschlussklappe abgeschwenkte Stellung vorgespannt, insbesondere mittels einer Feder vorgespannt. Dadurch ist sichergestellt, dass die Schließlasche beim Verschließen des Behälters von einem Nutzer besonders einfach und bedienerfreundlich korrekt zum Behälter positioniert werden und in Eingriff an die entsprechende Gegenstruktur am Behälterteil gebracht werden kann.

Bei einer Ausführungsform kann die Schließlasche einen Anschlag aufweisen, der in dem von der Verschlussklappe vollständig ausgeschwenkten Zustand an der Verschlussklappe anliegt. Dadurch ist sichergestellt, dass sich die Schließlasche bei offenem Verschluss stets in der gleichen Position befindet, was die Handhabbarkeit des Verschlusses weiter verbessert.

Bei einer Ausführungsform kann der Verschluss, insbesondere die Feder, eine Raststruktur, insbesondere einen Schlitz, aufweisen. Diese kann insbesondere zum Verrasten mit einer an dem einen Behälterteil angeordneten Gegenraststruktur, insbesondere in einer vollständig nach innen eingeklappten Position des Verschlusses, flach anliegend an das Behälterteil, genutzt werden, was zum Beispiel beim Reinigen und Waschen von besonderem Vorteil ist. Zum Beispiel kann der Verschluss am Behälterdeckel schwenkbar angeordnet sein und an der Deckelinnenseite die Gegenraststruktur angeordnet oder ausgebildet sein. Zum Waschen, insbesondere in einer Spül- oder Waschmaschine, kann der Verschluss nach innen an die Deckelinnenseite anliegend eingeklappt werden, wobei die Raststruktur mit der Gegenraststruktur in Eingriff gelangt und den Verschluss in dieser Reinigungsstellung hält. Dadurch kann die Handhabbarkeit des Verschlusses bzw. des Sterilbehälters bei einer Reinigung in vorteilhafter Weise verbessert werden.

Die Verschlussklappe kann einen Anschlag aufweisen, der bei in der Offenstellung befindlichem Verschluss an dem Behälterteil, an dem die Verschlussklappe angeordnet ist, zur Anlage kommt. Der Anschlag kommt vorzugsweise in Anlage, wenn der Verschluss, insbesondere die Verschlussklappe um einem Winkel von ca. 90° abgeschwenkt ist oder absteht. Dann kann der Verschluss in vorteilhafter Weise durch einen Nutzer als Griff genutzt werden, um den Behälterteil, an dem die Verschlussklappe angeordnet ist, handzuhaben.

Der Behälter kann ein Belüftungsventil zum Belüften eines von dem Behälterunterteil und dem Behälterdeckel umgebenen Behälterinnenraums aufweisen. Das Belüftungsventil kann mit dem Verschluss gekoppelt sein und in dessen Schließstellung geschlossen und in dessen Offenstellung geöffnet sein. Das Belüftungsventil kann insbesondere unter dem Verschluss, insbesondere unter der Schließlasche, angeordnet sein. Es ist dann für einen Nutzer nicht zugänglich und nicht manipulierbar, was Sterilität sicherstellt, solange der Sterilbehälter noch nicht geöffnet wurde. Zudem ist das Belüftungsventil bei geschlossener Verschlussvorrichtung/ Schließlasche hinter dieser verborgen und nicht sichtbar, was einen weiteren Schutz gegen Manipulierung darstellt.

Es ist von besonderem Vorteil, wenn die sich in ihrer Schließstellung befindliche Schließlasche mit dem Ventilkörper, insbesondere mit dem Ventilkörperkopf, in Eingriff steht und den Ventilkörper in die geschlossene Ventilstellung drängt. Auf diese Weise ist eine direkte Betätigung des Belüftungsventils in Abhängigkeit von der jeweiligen Funktionsstellung der Verschlussvorrichtung bzw. der Schließlasche gegeben.

Es ist von besonderem Vorteil, wenn die Riegelvorrichtung bei in der Schließstellung befindlicher Schließlasche mit dem Ventilkörper, insbesondere mit dem Ventilkörperkopf, in Eingriff steht und den Ventilkörper in die geschlossene Ventilstellung drängt. Hier wird das Belüftungsventil direkt von der Riegelvorrichtung betätigt. Es kann auch vorgesehen sein, dass die Riegelvorrichtung mit dem Ventilkörper, insbesondere mit dem Ventilkörperkopf, in Eingriff steht und diesen in die geschlossene Ventilstellung drängt, wenn sich die Riegelvorrichtung in der die Schließlasche verriegelnden Stellung befindet.

Es ist des Weiteren vorteilhaft, dass das Belüftungsventil bei einem Verschließen des Sterilbehälters mit dem Behälterdeckel automatisch geschlossen werden kann, so dass keine gesonderte Manipulation des Belüftungsventils nach dem Schließen des Behälters erforderlich ist, um Dichtheit und damit Sterilität zu gewährleisten. Wird der Behälterdeckel auf den Behälterunterteil aufgesetzt und der Verschluss geschlossen, wird das Ventil automatisch oder zwangsweise durch die Verschlussvorrichtung bzw. die Schließlasche betätigt und gegen seine Vorspannung in den geschlossenen, die Durchgangsöffnung abdichtenden Zustand gebracht. Verrastet der Verschluss mit der entsprechenden Gegenraststruktur, ist das Belüftungsventil geschlossen. Wird der Verschluss wieder geöffnet, öffnet sich das Belüftungsventil aufgrund seiner Vorspannung in die offene Stellung automatisch.

Durch die Verknüpfung der Belüftungsventilfunktion und der Funktion der Verschlussvorrichtung entsteht ein Sicherheits-Belüftungsventil, das nur dann belüftet, wenn der Sterilbehälter bewusst geöffnet wird. Ein unbeabsichtigtes Öffnen bzw. Betätigen des Ventils ist ausgeschlossen, und die Sterilität des Containers ist bis zur Deckelöffnung gewährleistet.

Ein Verschluss nach der Erfindung kann des Weiteren eine laschenartige Behälterbefestigung, insbesondere Deckelbefestigung, aufweisen, mit der der Verschluss am Behälter bzw. am Deckel anzuordnen und/oder zu befestigen ist. Vorzugsweise ist die Verschlussklappe relativ zur Behälterbefestungslasche schwenkbar, insbesondere um eine zwischen Verschlussklappe und Behälterbefestungslasche angeordnete Achse.

Die eingangs genannte Aufgabe wird auch durch einen medizinischen Sterilbehälter mit einem Verschluss in einer der vorstehend beschriebenen Ausführungsformen, insbesondere durch einen Sterilbehälter nach einem der angehängten Ansprüche gelöst.

Anders ausgedrückt betrifft die Erfindung in einer besonderen Ausführungsform einen Verschluss nach dem Kniehebel-Prinzip. Er kann über eine Deckelbefestigung (oder Deckellasche) am Behälterdeckel angebracht und über eine Achse schwenkbar mit diesem verbunden sein. Der Verschluss besteht im Wesentlichen aus der Verschlussklappe, der Verschlussachse, der Schließlaschenachse, der Schließlasche, Druckfedern zum Vorspannen des Riegelelements, dem Riegelelement und der Verschlussfeder. Die Verschlussklappe nimmt beide Achsen sowie das Riegelelement inkl. den zwei Druckfedern auf. Über die Verschlussachse ist die Verschlussklappe mit der Deckelbefestigung verbunden. Die Klappachse verbindet den Verschlussschnapper mit der Schließlasche. Das Riegelelement nimmt die beiden Druckfedern auf und ist auf die Verschlussklappe aufgesteckt. Über die Verschlussachse ist die Verschlussfeder zwischen Verschlussklappe und Verschlussschnapper montiert. Der Verschluss kann Kräfte über die Deckelbefestigung, Verschlussachse, Verschlussklappe, Schließlaschenachse und Schließlasche auf die Behälterwand, insbesondere auf eine Behälterblende, übertragen. Das Riegelelement greift in die Behälterwand, insbesondere in eine daran angeordnete Trägerplatte ein, überträgt jedoch keine Kräfte.

Um sicherzustellen, dass die Schließlasche immer in der richtigen Position ("ausgeklappt" zur Verschlussklappe) steht und damit gewährleistet ist, dass sie immer in der richtigen Position in die Behälterwand/Blende eingreift, wird sie über die Verschlussfeder in die korrekte Position gedrückt. Wird der Verschluss geschlossen, wird die Verschlussfeder zusammengepresst. Wird der Verschluss wieder geöffnet, so drückt die Verschlussfeder die Schließlasche sofort zurück in Richtung der Ausgangsposition. Die Schließlasche kann dabei geometrisch so ausgebildet sein, dass sie einen Anschlag gegen die Verschlussklappe ausbildet und somit immer in die richtige Position "ausfedert".

Ist der Verschluss geöffnet, so kann dieser als Halteelement, insbesondere als Halteelement am Deckel verwendet werden. Hierzu wird der Verschluss geöffnet und in eine zum Deckel waagerechte Position gebracht. Der Verschluss verfügt über einen geometrisch ausgebildeten Anschlag, mit dem die Verschlussklappe gegen die Deckelbefestigung anschlägt. Der Verschluss bildet damit ein festes Halteelement aus, ein sicheres Abheben des Deckels ist gewährleistet.

Zum Waschen oder Reinigen kann der Verschluss waagerecht zum Deckel "nach innen" eingeklappt werden und in dieser Position verrasten. Hierzu rastet die Verschlussfeder in die Deckellasche ein. Sie kann hierzu im oberen Bereich geschlitzt gestaltet sein. Die Deckellasche kann als Gegenstück zwei Rastnasen aufweisen, hinter die die Verschlussfeder schnappt. Zum Verrasten des Verschlusses wird dieser geöffnet und die geschlitzte Verschlussfeder rutscht, die Federenden sich zur Mitte hin zusammenbiegend, über die Rastnasen in die Rastposition. Über einen Formschluss zwischen Verschlussfeder und Deckellasche kann der Verschluss in der waagerechten Position zum Deckel gehalten werden. Wird der Verschluss aus der Rastposition entfernt, kann die Verschlussfeder wieder auf selbigen Weg aus der Verrastung gezogen werden.

Wird der Verschluss geschlossen, so greift der Verschlussschnapper in die Behälterwand oder Blende ein und "läuft/rutscht" darin vom eingeklappten Zustand zum geschlossenen Zustand. Hierzu ist der Verschluss so ausgebildet, dass die Schließlasche beim Schließen des Verschlusses näher zur Verschlussklappe einfedert und den "Winkel" zur Behälterwand/Blende verändert. Hierbei wird beim Schließen eine zur Verschlussklappe parallele Stellung der Schließlasche überfahren. Nach Überfahren dieser parallelen Stellung schnappt der Verschluss selbstständig vollends ein.

Im geschlossenen Zustand kann der Verschluss durch das Riegelelement arretiert werden. Hierzu verrastet das Riegelelement in der Behälterwand, insbesondere in einer daran angeordneten Trägerplatte. Zum Verrasten kann der Verschluss durch einen Nutzer einfach geschlossen oder anders ausgedrückt zu gedrückt werden. Über eine schräge Ebene kann der Verschluss "nach oben" gedrückt werden, bis er in die Behälterwand oder die Trägerplatte eingreifen kann. Durch die Druckfedern, die in das Riegelelement eingesteckt sind und die sich in der Verschlussklappe abstützen, kann das Riegelelement nach dem Eingreifen in den Ausschnitt der Trägerplatte sofort wieder "nach unten" gedrückt werden. Der Verschluss ist durch den geometrischen Hinterschnitt des Riegelelements arretiert.

Als Schutz gegen ungewolltes Öffnen kann es erforderlich sein, zum Öffnen des Verschlusses das Riegelelement aktiv zu betätigen, um seine Verrastung in der Behälterwand/Trägerplatte zu lösen. Hierzu kann die Verschlussverriegelung durch den Nutzer "nach oben" gedrückt werden. Als zweite Sicherung gegen ungewolltes Öffnen kann es erforderlich sein, den nun entsperrten Verschluss aktiv aus dem eingeschnappten Zustand zu lösen. Ein alleiniges Öffnen der Verschlussverriegelung kann zum Öffnen des Verschlusses nicht ausreichend sein.

Der Verschluss kann so ausgebildet sein, dass er im geschlossenen und verriegelten Zustand die Plombe aufnehmen kann. Hierzu kann die Verschlussklappe eine Öffnung zum Durchstecken der Plombe aufweisen. Eine Verplombung eines nicht korrekt geschlossenen Behälters kann unmöglich sein. Ist der Verschluss nicht geschlossen bzw. das Riegelelement nicht korrekt mit dem Behälterunterteil verrastet, steht der Verschluss so weit von dem Behälterunterteil ab, dass die eingesteckte Plombe nicht verplombt werden kann. Wird die Plombe vor dem Verschließen des Verschlusses angebracht, so lässt sich der Verschluss nicht mehr schließen. Ist der Verschluss verschlossen und mit einer Plombe gesichert, so kann der Verschluss nur durch die Zerstörung der Plombe geöffnet werden. Hierzu kann der Verschluss durch das Betätigen des Riegelelements entsperrt und geöffnet werden. Im Öffnungsvorgang kann die Verschlussklappe in die verrastete Plombe eingreifen und diese durch das zerstörende Aufbiegen der Plombenflügel zerstören. Des Weiteren kann der Verschluss so ausgebildet sein, dass eine Entfernung der angebrachten Plombe ausschließlich zerstörend möglich ist. Ist die Plombe montiert, kann sie in einer Aussparung des Riegelelements versenkt sein und bündig dazu abschließen. Ein Verdrehen der Plombe, wie zur Entfernung erforderlich, kann geometrisch bedingt nicht möglich sein.

Weitere Merkmale und Vorteile der vorliegenden Erfindung ergeben sich aus der folgenden beispielhaften Beschreibung der Erfindung sowie einer besonders bevorzugten Ausführungsform anhand von Figuren. Diese sind lediglich schematischer Natur und dienen nur dem Verständnis der Erfindung. Dabei zeigt:
Fig. 1 einen Verschluss für einen medizintechnischen Sterilbehälter gemäß einer ersten Ausführungsform der Erfindung in einer perspektivischen Explosionsdarstellung;
Fig. 2 den Verschluss der Fig. 1 im montierten Zustand in einer schematischen perspektivischen Darstellung;
Fig. 3 eine Teilschnittansicht der Fig. 2;
Fig. 4 eine weitere Teilschnittansicht der Fig. 2;
Fig. 5 einen erfindungsgemäßen Verschluss, angeordnet an einem Sterilbehälter, in einer Schnittdarstellung in der Schließstellung;
Fig. 6 den Verschluss der Figur 5 in einer gegenüber der Schließstellung um einen Winkel von 3° geöffneten Stellung;
Fig. 7 den Verschluss der Figur 5 in einer gegenüber der Schließstellung um einen Winkel von 23° geöffneten Stellung;
Fig. 8 den Verschluss der Figur 5 in einer gegenüber der Schließstellung um einen Winkel von 34° geöffneten Stellung;
Fig. 9 einen Verschluss für einen medizintechnischen Sterilbehälter gemäß einer zweiten Ausführungsform der Erfindung in einer schematischen perspektivischen Darstellung;
Fig. 10 den Verschluss der Fig. 9 in einer Seitenansicht;
Fig. 11 eine Stirnansicht der Fig. 9;
Fig. 12 eine Rückansicht des Verschlusses der Fig. 9;
Fig. 13 eine Vorderansicht des Verschlusses der Fig. 9; und
Fig. 14 einen Verschluss für einen medizinischen Sterilbehälter gemäß einer dritten Ausführungsform der Erfindung in einer schematischen Darstellung.

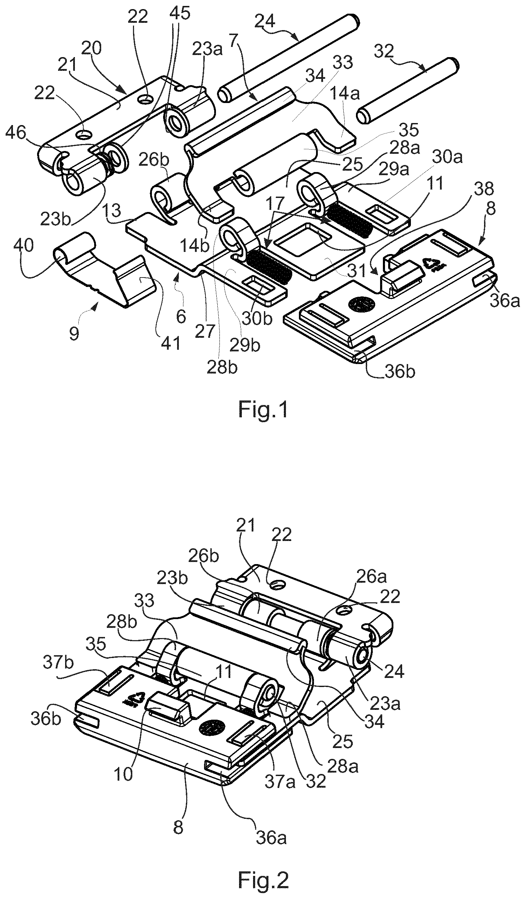

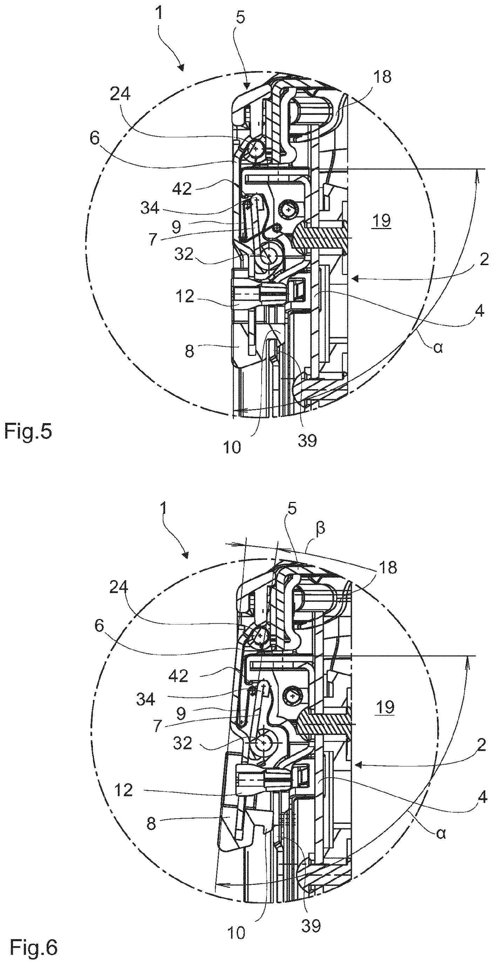

In der Figur 1 ist ein Verschluss für einen medizintechnischen Sterilbehälter 1 nach der Erfindung in einer perspektivischen Explosionsdarstellung gezeigt. In den Schnittdarstellungen der Figuren 5 bis 8 ist der Verschluss in Schnittansichten dargestellt, wobei Teile des Sterilbehälters 1 zu erkennen sind. Dieser weist einen Behälterunterteil 2 und einen Deckel 5 als Behälterteile auf. Der Behälterunterteil 2 besitzt einen Behälterboden 3 und eine Behälterwand 4. Auf der vom Behälterboden 3 abgewandten Seite der Behälterwand 4 ist der Behälterdeckel 5 anzuordnen oder angeordnet. Der Behälterdeckel 5 ist gegenüber der Behälterwand 4 mit einer Dichtung 18 abgedichtet, so dass bei verschlossenem Sterilbehälter 1 dessen Innenraum 19 gegenüber der Umgebung hermetisch abgedichtet ist, mit Ausnahme ggf. vorhandener Filter und Filteröffnungen, die in den Figuren jedoch nicht gezeigt sind.

Am Behälterdeckel 5 ist eine Deckellasche 20, insbesondere mittels Nieten oder Schrauben, angeordnet. Die Deckellasche 20 ist ein Blechformteil und perspektivisch in Figur 1 gezeigt. Sie besteht im Wesentlichen aus einer Grundplatte 21, in der zwei Löcher 22 zur Aufnahme von Befestigungsschrauben oder -nieten ausgebildet sind. An der im am Deckel 5 befestigten Zustand unteren Seite weist die Grundplatte 21 zwei Laschen auf, die zu einer linken Lageraufnahme 23a und einer rechten Lageraufnahme 23b ausgebildet sind. Die Lageraufnahmen 23a,b dienen der Aufnahme einer Verschlussachse 24, die aus einem zylinderförmigen Achsstift gebildet ist.

Mittels der Verschlussachse 24 ist eine Verschlussklappe 6 um die Achse 24 schwenkbar an der Deckellasche 20 und damit am Deckel 5 angeordnet. Die Verschlussklappe 6 ist ebenfalls als Blechformteil ausgebildet. Sie besteht im Wesentlichen aus einer langgestreckt z-förmigen Grundplatte 25, an der endseitig zwei Laschen zu einer linken Lageraufnahme 26a und einer rechten Lageraufnahme 26b ausgebildet sind. Seitlich außerhalb der Lageraufnahmen 26a,b ist jeweils ein Anschlag 13 durch endseitige Vorsprünge der Grundplatte 25 ausgebildet. Diese Anschläge 13 liegen bei vollständig vom Deckel 5 abgeschwenktem Verschluss an der Deckellasche 20 an und definieren einen maximalen Schwenkwinkel a, um den die Verschlussklappe 6 relativ zur Ebene des Deckels 5 abgeschwenkt werden kann.

Zwischen den Lageraufnahmen 23a,b der Deckellasche 20 und den Lageraufnahmen 26a,b der Verschlussklappe 6 befinden sich auf der Achse 24 jeweils eine Teflonscheibe 45. Auf der einen (in der Fig. 1 linken) Lageraufnahmeseite befindet sich zwischen der linken Lageraufnahme 23a und der entsprechenden Teflonscheibe 45 zusätzlich eine multigewellte Federscheibe 46. Die multigewellte Federschreibe 46 übt eine axiale Kraft zwischen den beiden Lageraufnahmen 23a und 26a aus und stellt über die Teflonscheiben 45 eine mechanische Hemmung zwischen der Deckellasche 20 und der Verschlussklappe 6 bzw. zwischen dem Deckel 5 und dem Verschluss her. Hiermit wird eine dauerhafte "Schwergängigkeit" des Verschlusses erreicht. Ein "Herumschlabbern" des Verschlusses wird verhindert. Durch die Hemmung ist der Verschluss zur Reinigung "einklappbar". Die Gewichtskraft des Verschlusses wird somit gehalten. Der Einsatz der multigewellten Federscheibe 46 sowie der Teflonscheibe 45 stellt eine verschleißfreie Möglichkeit zur Hemmung dar. Bekannte Lösungen wie kegelige Achsen, gebogene Achse, geschränkte Lageraufnahmen sowie eine einseitige Einpressung der Achse in die Lageraufnahme sind stark reibungsbehaftet und verlieren über die Lebenszeit ihre Hemmung. Alternativ kann anstelle einer Teflonscheibe 45 eine Scheibe aus Edelstahl oder Kunststoff verwendet werden.

Etwa mittig der Verschlussklappe 6 besitzt diese eine Stufe 27. Auf der den Lageraufnahmen 26a,b gegenüberliegenden Seite der Stufe 27 sind zwei zueinander parallele Streifen aus der Grundplatte 25 ausgeschnitten und umgebogen, so dass sie zwei Lageraufnahmen 28a,b ausbilden. Die Lageraufnahmen 28a,b und die Lageraufnahmen 26a,b sind auf einer bzw. auf derselben Seite der Grundplatte 25 angeordnet. In den durch das Umbiegen der Streifen für die Lageraufnahmen 28a,b entstandenen Freiräume sind jeweils eine Feder 17, hier eine Spiralfeder 17, angeordnet. Seitlich außerhalb der die Federn 17 aufnehmenden Freiräume ist die Grundplatte 25 zu Seitenlaschen 29a,b ausgebildet. In jede Seitenlasche 29a,b ist eine Ausnehmung 30a bzw. 30b eingebracht. Die Ausnehmungen 30a,b dienen, wie später genauer ausgeführt wird, einer in Richtung der Grundplatte 25 verrückbaren Anordnung und einem Halten eines Riegelelements 8. Zwischen den Lageraufnahmen 28a,b ist die Grundplatte 25 zu einer Mittellasche 31 ausgebildet. Diese weist mittig eine Ausnehmung 11 auf, die der Aufnahme einer Plombe 12 zum Verplomben des Sterilbehälters 1 dient. Die Plombe 12 ist in den Figuren 5 und 6 zu erkennen und in DE 10 2012 004 961 A1 detailliert beschrieben.

Mittels der Lageraufnahmen 28a,b ist eine Schließlasche 7 schwenkbar an der Verschlussklappe 6 angeordnet. Dazu ist in den Lageraufnahmen 28a,b eine Schließlaschenachse 32 aufgenommen, die aus einem zylinderförmigen Achsstift gebildet ist. Die Schließlasche 7 ist als Blechbauteil ausgebildet. Sie besteht im Wesentlichen aus einer Grundplatte 33, die endseitig zu einer verstärkten Eingriffszunge 34 umgebogen ist. An ihrem der Eingriffszunge 34 gegenüberliegenden Ende ist die Schließlasche 7 in einem mittleren Abschnitt zu einer Lagerachsenaufnahme 35 umgebogen. Die Lagerachsenaufnahme 35, die Schließlaschenachse 32 und die Lageraufnahmen 28a,b bilden ein Schwenklager aus, mit dem die Schließlasche 7 schwenkbar zur Verschlussklappe 6 an dieser angeordnet ist. Seitlich außerhalb der Lagerachsenaufnahme 35 weist die Schließlasche 7 zwei Anschläge 14a,b auf, die einen Schwenkwinkel β zwischen der Schließlasche 7 und der Verschlussklappe 6 begrenzen. Die Verschlussklappe 6 und die Schließlasche 7 bilden zusammen mit den Schwenkachsen 24 und 32 einen Kniehebelmechanismus aus.

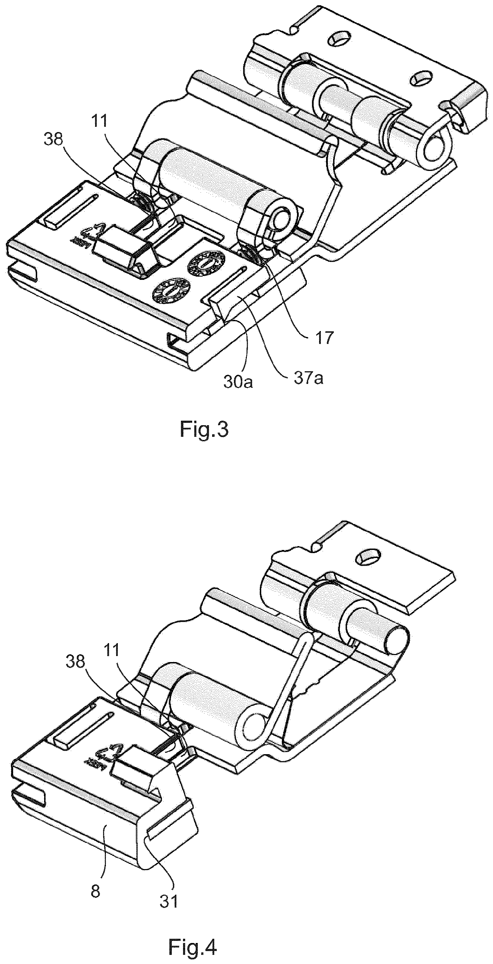

Der Verschluss weist des Weiteren das Riegelelement 8 auf. Dieses ist im dargestellten Ausführungsbeispiel als Kunststoffspritzgussteil ausgebildet. Das Riegelelement 8 besitzt zwei seitliche, in Längsrichtung des Verschlusses verlaufende Ausnehmungen 36a,b, die einer Aufnahme der beiden Seitenlaschen 29a,b der Verschlussklappe 6 dienen. Im Bereich jeder schlitzförmigen Ausnehmung 36a,b ist ein Federelement (eine Federzunge) 37a,b angeordnet, das in die jeweilige Ausnehmung 30a,b der entsprechenden Seitenlasche 29a,b eingreift (siehe Fig. 2 und Fig. 3). Das Riegelelement 8 ist gegenüber der Verschlussklappe 6 in der Längsrichtung des Verschlusses verrückbar. Die in die Ausnehmungen 30a,b eingreifenden Federelemente 37a,b bilden dabei in der geöffneten Verschlussstellung, d.h. wenn der Verschluss nicht im Behälterunterteil 2 bzw. in der Trägerplatte eingerastet ist, einen ersten, unteren Anschlag aus, gegen den das Riegelelement 8 federbelastet (Federn 17) gedrückt wird und der eine Endposition des Riegelelements 8 an der Verschlussklappe 6 definiert (siehe Figur 3). Die Bewegung des Riegelelements 8 in die entgegengesetzte Richtung, d.h. entgegen den Federkräften der Federn 17, wird durch einen zweiten, oberen Anschlag begrenzt, der durch einen mittigen Abschnitt des Riegelelements 8 gebildet wird, der gegen eine Stirnkante der Mittellasche 31 anschlägt (siehe Figur 4).

Zum Zwecke einer Verriegelung des Verschlusses mit dem Sterilbehälter 1 weist das Riegelelement 8 eine Sperrklinke 10 auf, die mit einer Rastkante 39 des Behälterunterteils 2 verhaken kann. In der geschlossenen Verschlussstellung, d.h. wenn der Verschluss im Behälterunterteil 2 eingerastet ist, bilden nicht die Federelemente 37a,b den unteren Anschlag, sondern die Sperrklinke 10, die in der geschlossenen Verschlussstellung an der Rastkante 39 des Behälterunterteils 2 anschlägt (siehe Figur 5).

Liegt die Sperrklinke an der Rastkante 39 des Behälterunterteils 2 an, befindet sich das Riegelelement 8 in einer Riegelposition, in der es den Verschluss gegenüber dem Sterilbehälter 1 in dessen Schließstellung verriegeln kann. Liegt der mittige Abschnitt des Riegelelements 8 an der Stirnkante der Mittellasche 31 der Verschlussklappe 6 an, befindet sich das Riegelelement 8 in einer Freigabeposition, in der es von der Deckellasche 20 minimal entfernt ist und den Verschluss gegenüber dem Sterilbehälter 1 aus dessen Schließstellung entriegeln kann.

Das Riegelelement 8 weist eine mittige Ausnehmung 38 auf. Diese ist hinsichtlich ihrer Größe und Position auf die in der Verschlussklappe 6 vorgesehene Ausnehmung 11 derart abgestimmt, dass die Ausnehmung 11 in der Verschlussklappe 6 nicht durch das Riegelelement 8 verdeckt wird, weder wenn sich das Riegelelement 8 in der Riegelposition befindet, noch wenn sich das Riegelelement 8 in der Freigabeposition befindet (siehe Figur 3 und Figur 4).

Bei in der Riegelposition befindlichem Riegelelement 8 kann in diesem eine Plombe 12 angeordnet werden. Dies ist nicht möglich, wenn das Riegelelement 8 aus der Riegelposition verrückt wurde. Ist eine Plombe 12 in den Ausnehmungen 11 und 38 angeordnet und der Sterilbehälter 1 damit verplombt, ist die Plombe durch den Verschluss, insbesondere durch das Riegelelement 8 derart aufgenommen, dass sie für einen Nutzer nicht zugänglich ist und nicht ohne Verschieben des Riegelelements 8 und Öffnen des Verschlusses entfernt werden kann. Des Weiteren wird die Plombe 12 durch Öffnen des Verschlusses zwangsläufig zerstört, da die Verschlussklappe 6 in die verrastete Plombe eingreift und die Plombenflügel zerstörend aufbiegt.

Die Verschlussklappe 6 und die Schließlasche 7 sind mittels einer Verschlussfeder 9 gegeneinander vorgespannt. Die Verschlussfeder 9 ist ein Biegeteil oder Blechformteil aus einem Federstahl und ist endseitig zu einer Lagerachsenaufnahme 40 umgebogen. Sie ist mittels dieser an der Verschlussachse 24 angeordnet. An ihrer der Lagerachsenaufnahme 40 gegenüberliegenden Seite ist die Verschlussfeder um mehr als 90° umgebogen und bildet einen Federarm 41 aus. Die Verschlussfeder 9 liegt mit dem zwischen der Lagerachsenaufnahme 40 und dem Federarm 41 befindlichen Abschnitt an der Seite der Riegellasche 7 an der Verschlussklappe 6 an und ist zwischen dieser und der Schließlasche 7 angeordnet. Der Federarm 41 ist an der Schließlasche 7 abgestützt, so dass die Verschlussfeder 9 die Schließlasche 7 in eine von der Verschlussklappe 6 abgeschwenkte Lage vorspannt. Die Schließlasche 7 ist gegen die Vorspannung der Verschlussfeder 9 in Richtung der Verschlussklappe 6 schwenkbar.

Die Figuren 5 bis 8 zeigen den Verschluss in Schnittdarstellungen in unterschiedlichen Stellungen relativ zum Sterilbehälter 1, um die Funktionsweise des Verschlusses zu verdeutlichen. Figur 5 zeigt den Verschluss mit der Verschlussklappe 6 in der Schließstellung. Die Verschlussklappe 6 liegt seitlich an der Behälterwand 4 an. Die Eingriffszunge 34 hintergreift eine Rastkante 42 des Behälterunterteils 2, so dass der Deckel 5 in Richtung der Behälterwand 4 gezogen wird und dichtend an der Dichtung 18 anliegt. Die Sperrklinke 10 des Riegelelements 8 hintergreift in der Schließstellung die Rastkante 39. Das Riegelelement 8 befindet sich in der Riegelposition. Die Plombe 12 durchgreift die beiden Ausnehmungen 11 und 38 und ist in diesen für einen Nutzer nicht zugänglich aufgenommen.

Zum Öffnen des Verschlusses ist durch einen Nutzer das Riegelelement 8 gegen die Vorspannung der Druckfedern 17 in Richtung der Deckellasche 20 (in Figur 5 nach oben) zu verrücken. Die Sperrklinke 10 löst sich von der Rastkante 39 und die Verschlussklappe kann um die Verschlussachse 24 von der Behälterwand 4 abgeschwenkt werden. Dabei wird die in den Ausnehmungen 11, 38 befindliche Plombe zerstört. Infolge der kniehebelartigen Ausbildung des Verschlusses schwenkt die Schließlasche 7 dabei von der Verschlussklappe 6 ab. Figur 6 zeigt den Verschluss kurz vor der Zerstörung der Plombe 12.

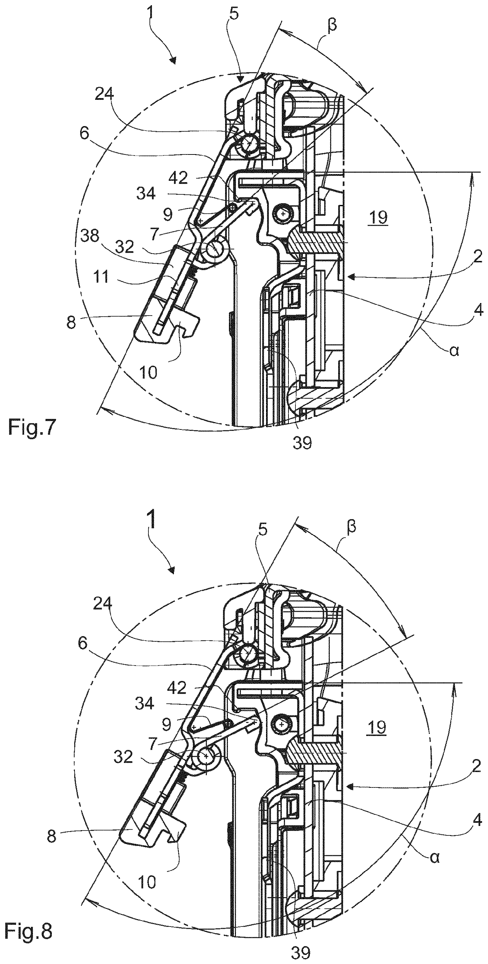

Figur 7 zeigt den Verschluss in einer weiter von der Behälterwand 4 abgeschwenkten Lage als Figur 6. Die Eingriffszunge 34 liegt noch immer hinter der Rastkante 42 an. Die Plombe 12 ist zerstört und entfernt. Im Vergleich der Figuren 6 und 7 erkennt man gut, wie die Verschlussfeder 9 mit größerem Schwenkwinkel α entspannt. Figur 8 zeigt den Verschluss schließlich in einer Lage, in der sich die Eingriffszunge 34 vom Behälterunterteil 2 gelöst hat. Insbesondere Figur 7 ist zu entnehmen, dass eine Plombe nicht zu befestigen und zu verplomben ist, wenn sich der Verschluss zwar in Eingriff mit Behälterunterteil 2 und Deckel 5 befindet, der Verschluss jedoch nicht vollständig geschlossen ist (Figur 5). Ein Verplomben ist nur in der Stellung der Figur 5 möglich.

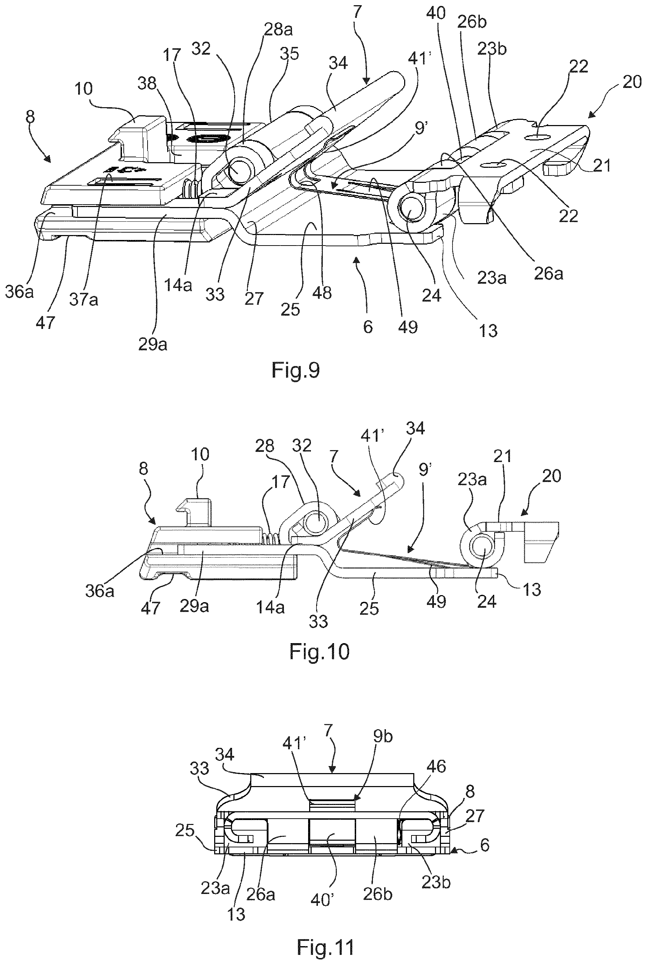

Eine zweite Ausführungsform eines erfindungsgemäßen Verschlusses für einen medizintechnischen Sterilbehälter wird anhand der Figuren 9 bis 12 erläutert, wobei im Folgenden nur auf die Unterschiede zu der ersten Ausführungsform eingegangen wird und bezüglich gleicher Merkmale und Funktionen auf die Beschreibung der vorherigen Beschreibung der ersten Ausführungsform verwiesen wird.

Die zweite Ausführungsform unterscheidet sich von der ersten Ausführungsform im Wesentlichen nur in der Gestaltung des Riegelelements 8' und der Verschlussfeder 9'.

Das Riegelelement 8' weist zusätzlich an seiner Außenseite bzw. an der im montierten Zustand vom Behälter abgewandten Seite eine waagrechte bzw. parallel zu den Achsen 24 und 32 verlaufende Quernut 47 auf, welche die Handhabung des Riegelelements 8' beim Verschieben relativ zur Verschlussklappe 6 und entgegen der Federkraft der Federn 17 erleichtert.

Der an der Schließlasche 7 abgestützte Federarm 41' der Verschlussfeder 9' weist eine mittige Ausnehmung 48 auf. Der zwischen der Lagerachsenaufnahme 40 und dem Federarm 41' befindliche Abschnitt der Verschlussfeder weist eine Federzunge 49 auf, welche federvorgespannt an der Oberfläche der Grundplatte 25 der Verschlussklappe 6 anliegt (siehe Figur 10). Wie bei der ersten Ausführungsform ist der Federarm 41' der Schließlasche 7 abgestützt, so dass die Verschlussfeder 9' die Schließlasche 7 in eine von der Verschlussklappe 6 abgeschwenkte Lage vorspannt. Die Schließlasche 7 ist gegen die Vorspannung der Verschlussfeder 9' in Richtung der Verschlussklappe 6 schwenkbar. Dabei bleibt die Federzunge 49 ständig in Kontakt mit der Grundplatte 25.

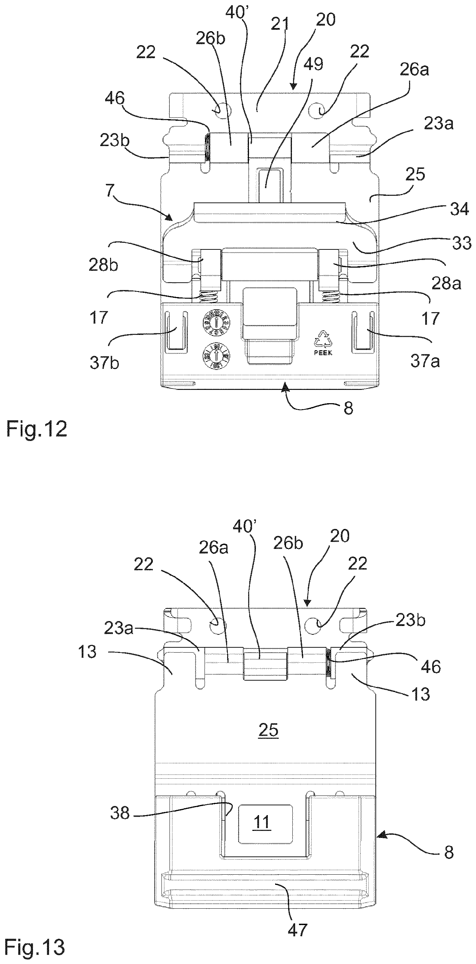

Ein weiterer Unterschied zur ersten Ausführungsform ist aus den Figuren 11 und 12 erkennbar. Bei der zweiten Ausführungsform befinden sich zwischen den Lageraufnahmen 23a,b der Deckellasche 20 und den Lageraufnahmen 26a,b der Verschlussklappe 6 keine Teflonscheiben 45. Auf einer Lageraufnahmeseite ist die multigewellte Federscheibe 46 unmittelbar zwischen den linken Lageraufnahmen 23b und 26b sandwichartig zwischengeschaltet.

Um die mechanische Hemmung bzw. die gewollte Schwergängigkeit des Verschlusses weiter zu erhöhen, können an oder zwischen den Lageraufnahmen 23a,b und 26a,b Reibbeläge vorgesehen sein.

Die Federscheibe 46 kann als eine einstellbare Federscheibe ausgestaltet sein, um die mechanische Hemmung bzw. die gewünschte Schwergängigkeit den jeweiligen Anforderungen anpassen zu können und ggf. bei längerem Gebrauch nachjustieren zu können.

Figur 11 zeigt eine Rückansicht, d.h. die dem Behälter zugewandte Seite, des Verschlusses und Figur 12 zeigt eine Vorderansicht, d.h. die Außenseite bzw. die dem Benutzer zugewandte Seite des Verschlusses.

Im Übrigen entsprechen die Funktionsweise und die Wirkung der zweiten Ausführungsform denen der ersten Ausführungsform.

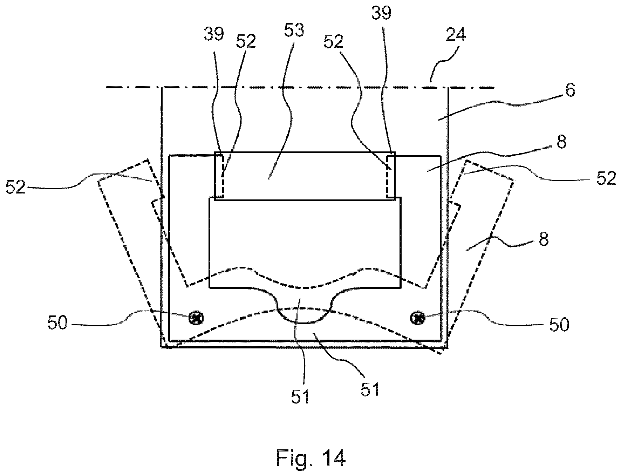

Figur 14 zeigt einen Verschluss für einen medizinischen Sterilbehälter gemäß einer dritten Ausführungsform der Erfindung in einer schematischen Darstellung. Dabei ist das Riegelelement 8 rotatorisch verrückbar an der Verschlussklappe 6 angeordnet. Das Riegelelement 8 und die Verschlussklappe 6 sind schwenkbar um die Verschlussachse 24 an einem nicht dargestellten ersten Behälterteil vorgesehen. Das Riegelelement 8 ist in Figur 14 in einem ausgerückten, nach außen geschwenkten Zustand und in einem eingerückten, eingreifenden Zustand dargestellt. In dem eingerückten Zustand greifen Riegelvorsprünge 52, welche an dem Riegelelement 8 vorgesehen sind, in Rastkanten 39 eines Eingriffsabschnitts 53 eines nicht dargestellten zweiten Behälterteils ein. Die Riegelvorsprünge 52 können dabei insbesondere auch schräge Auflaufflächen aufweisen. Vorzugsweise bildet sich in einem verriegelten Zustand des Riegels ein Hinterschnitt zwischen den Riegelvorsprüngen 52 und dem Eingriffsabschnitt 53 aus. Das Riegelelement 8 kann relativ zu der Verschlussachse um zwei Drehpunkte 50, einem rechtsseitigen Drehpunkt 50 und einem linksseitigen Drehpunkt 50, rotiert bzw. nach außen und nach innen geschwenkt werden. In einem mittleren Abschnitt zwischen den Drehpunkten 50 ist ein elastischer Abschnitt 51 des Riegelelements 8 vorgesehen, der bei einem Schwenken nach außen des Riegelelements 8 gedehnt wird und bei einem Schwenken nach innen des Riegelelements 8 wieder in seinen ungedehnten Ausgangszustand übergeht. Anstatt der festen Drehpunkte 50 können auch Langlöcher an den Seiten vorgesehen sein und anstatt des elastischen Abschnitts 51 kann auch ein Gelenk in dem mittleren Bereich des Riegelelements 8 vorgesehen sein.

### Bezugszeichenliste

- 1: Sterilbehälter
- 2: Behälterteil, Behälterunterteil
- 3: Behälterboden
- 4: Behälterwand
- 5: Behälterteil, Behälterdeckel
- 6: Verschlussklappe
- 7: Schließlasche
- 8: Riegelelement
- 9, 9': Feder, Verschlussfeder
- 10: Sperrklinke
- 11: Ausnehmung
- 12: Plombe
- 13: Anschlag
- 14: Anschlag
- 17: Feder (für das Riegelelement 8)
- 18: Dichtung
- 19: Innenraum des Sterilbehälters
- 20: Deckellasche
- 21: Grundplatte
- 22: Loch
- 23a,b: Lageraufnahme
- 24: Verschlussachse
- 25: Grundplatte
- 26a,b: Lageraufnahme
- 27: Stufe
- 28a,b: Lageraufnahme
- 29a,b: Seitenlasche
- 30a,b: Ausnehmung
- 31: Mittellasche
- 32: Schließlaschenachse
- 33: Grundplatte
- 34: Eingriffszunge
- 35: Lagerachsenaufnahme
- 36a,b: Ausnehmung
- 37: Federelement
- 38: Ausnehmung
- 39: Rastkante
- 40: Lagerachsenaufnahme
- 41, 41': Federarm
- 42: Rastkante
- 45: Teflonscheibe
- 46: multigewellte Federscheibe
- 47: Quernut
- 48: Ausnehmung
- 49: Federzunge
- 50: Drehpunkt
- 51: elastischer Abschnitt
- 52: Riegelvorsprung
- 53: Eingriffsabschnitt
- α: Schwenkwinkel
- β: Schwenkwinkel

## Patentansprüche

1. Verschluss für einen medizinischen Sterilbehälter (1) mit einem wannenartigen ersten Behälterteil (2) und einem deckelartigen zweiten Behälterteil (5), mit einer schwenkbar zwischen einer Schließstellung, in der die Behälterteile (2, 5) mittels des Verschlusses miteinander verriegelt sind, und einer Offenstellung, in der die Behälterteile (2, 5) unverriegelt sind, an einem der Behälterteile (2, 5) angeordneten Verschlussklappe (6), wobei an der Verschlussklappe (6) eine Schließlasche (7) als ein Kniehebel schwenkbar angeordnet ist, die ausgebildet ist, um in der Schließstellung des Verschlusses mit dem anderen Behälterteil (2, 5) in Eingriff zu stehen, **dadurch gekennzeichnet, dass**
der Verschluss ein Riegelelement (8) aufweist, das die Verschlussklappe (6) in der Schließstellung des Verschlusses mit dem anderen Behälterteil (2, 5) verriegelt, wobei das Riegelelement (8) zwischen einer den Verschluss verriegelnden Stellung und einer den Verschluss freigebenden Stellung verrückbar an der Verschlussklappe (6) angeordnet ist, und
das Riegelelement (8) eine Sperrklinke (10) aufweist, die in der Schließstellung des Verschlusses mit dem anderen Behälterteil (2, 5) verrastet ist.

2. Verschluss für einen medizinischen Sterilbehälter (1) nach Anspruch 1, **dadurch gekennzeichnet, dass** das Riegelelement (8) linear verrückbar an der Verschlussklappe (6) angeordnet ist.

3. Verschluss für einen medizinischen Sterilbehälter (1) nach Anspruch 1 oder 2, **dadurch gekennzeichnet, dass** das Riegelelement (8) relativ zur Verschlussklappe (6) zwischen einer Riegelposition, in der das Riegelelement (8) mit dem anderen Behälterteil (2, 5) verriegelt ist, und einer Freigabeposition, in der das Riegelelement (8) von dem anderen Behälterteil (2, 5) entriegelt ist, positionierbar ist.

4. Verschluss für einen medizinischen Sterilbehälter (1) nach einem der vorstehenden Ansprüche, **dadurch gekennzeichnet, dass** die Verschlussklappe (6) eine Ausnehmung (11) aufweist und das Riegelelement (8) eine Ausnehmung (38) aufweist, wobei bei in der Riegelposition befindlichem Riegelelement (8) eine Plombe (12) zum Verplomben des medizinischen Sterilbehälters (1) in den Ausnehmungen (11, 38) anordbar ist.

5. Verschluss für einen medizinischen Sterilbehälter (1) nach Anspruch 4, **dadurch gekennzeichnet, dass** die Plombe (12) im montierten Zustand in der Ausnehmung (38) des Riegelelements (8) versenkt ist und bündig dazu abschließt.

6. Verschluss für einen medizinischen Sterilbehälter (1) nach einem der vorstehenden Ansprüche, **dadurch gekennzeichnet, dass** die Schließlasche (7) in der Schließstellung des Verschlusses an der Verschlussklappe (6) anliegt und in einer Offenstellung des Verschlusses von der Verschlussklappe (6) abgeschwenkt ist.

7. Verschluss für einen medizinischen Sterilbehälter (1) nach einem der vorstehenden Ansprüche, **dadurch gekennzeichnet, dass** die Schließlasche (7) in der Schließstellung des Verschlusses durch das Riegelelement (8) zumindest abschnittsweise umgriffen wird.

8. Verschluss für einen medizinischen Sterilbehälter (1) nach einem der vorstehenden Ansprüche, **dadurch gekennzeichnet, dass** das Riegelelement (8) in die Riegelposition vorgespannt ist, insbesondere mittels einer Feder (17).

9. Medizinischer Sterilbehälter (1) mit einem Verschluss nach einem der vorstehenden Ansprüche.

## Claims

1. A closure for a medical sterile container (1) comprising a trough-like first container part (2) and a lid-type second container part (5), comprising a closure flap (6) which is arranged on one of the container parts (2, 5) so as to be able to pivot between a closed position, in which the container parts (2, 5) are interlocked with each other by means of the closure, and an open position in which the container parts (2, 5) are unlocked, a closure lug (7) as a toggle lever being pivotably arranged on the closure flap (6) and designed to engage the other container part (2, 5) in the closed position of the closure, **characterized in that**
the closure comprises a latch element (8) which in the closed position of the closure interlocks the closure flap (6) with the other container part (2, 5), the latch element (8) being movably arranged on the closure flap (6) between a position locking the closure and a position releasing the closure, and
the latch element (8) comprising a locking pawl (10) which can be locked in place with the other container part (2, 5).

2. The closure for a medical sterile container (1) according to claim 1, **characterized in that** the latch element (8) is arranged on the closure flap (6) so as to be able to be linearly moved.

3. The closure for a medical sterile container (1) according to claim 1 or claim 2, **characterized in that** the latch element (8) can be positioned relative to the closure flap (6) between a locked position, in which the latch element (8) is interlocked with the other container part (2, 5), and a release position, in which the latch element (8) is unlocked from the other container part (2, 5).

4. The closure for a medical sterile container (1) according to any of the preceding claims, **characterized in that** the closure flap (6) comprises a recess (11) and the latch element (8) comprises a recess (38), a seal (12) for sealing the medical sterile container (1) being able to be disposed in the recesses (11, 38) when the latch element (8) is in the locked position.

5. The closure for a medical sterile container (1) according to claim 4, **characterized in that** the seal (12), when mounted, is embedded in the recess (38) of the latch element (8) and is flush therewith.

6. The closure for a medical sterile container (1) according to any of the preceding claims, **characterized in that** the closure lug (7) rests against the closure flap (6) in the closed position of the closure and is swiveled away from the closure flap (6) in an open position of the closure.

7. The closure for a medical sterile container (1) according to any of the preceding claims, **characterized in that** the latch element (8) encompasses the closure lug (7) at least in section in the closed position of the closure.

8. The closure for a medical sterile container (1) according to any of the preceding claims, **characterized in that** the latch element (8) is prestressed in the locked position, in particular by means of a spring (17).

9. A medical sterile container (1) comprising a closure according to any of the preceding claims.

## Revendications

1. Fermeture pour un contenant médical stérile (1) comportant une première partie de contenant de type cuvette (2) et une seconde partie de contenant (5) de type couvercle, un volet de fermeture (6) agencé sur l'une des parties de contenant (2, 5), de façon à pouvoir pivoter entre une position de fermeture dans laquelle les parties de contenant (2, 5) sont verrouillées conjointement au moyen de la fermeture, et une position d'ouverture dans laquelle les parties de contenant (2, 5) sont déverrouillées, dans laquelle au niveau du volet de fermeture (6) une patte de fermeture (7) est agencée de façon à pouvoir pivoter en tant que levier coudé qui est conçu pour être en prise dans la position de fermeture de la fermeture avec l'autre partie de contenant (2, 5), **caractérisée en ce que**
la fermeture présente un élément de verrouillage (8) qui verrouille le volet de fermeture (6) dans la position de fermeture de la fermeture avec l'autre partie de contenant (2, 5), dans laquelle l'élément de verrouillage (8) est agencé au niveau du volet de fermeture (6) de façon à pouvoir être déplacé entre une position verrouillant la fermeture et une position libérant la fermeture,
et l'élément de verrouillage (8) présente un loquet (10) qui peut s'accrocher avec un bord d'encliquetage (39) d'une partie de contenant (2, 5), et
l'élément de verrouillage (8) présente un loquet (10) qui est encliqueté dans la position de fermeture de la fermeture avec l'autre partie de contenant (2, 5).

2. Fermeture pour un contenant médical stérile (1) selon la revendication 1, **caractérisée en ce que** l'élément de verrouillage (8) est agencé de façon à pouvoir être déplacé de façon linéaire au volet de fermeture (6).

3. Fermeture pour un contenant médical stérile (1) selon la revendication 1 ou 2, **caractérisée en ce que** l'élément de verrouillage (8) peut être positionné par rapport au volet de fermeture (6) entre une position de verrouillage dans laquelle l'élément de verrouillage (8) est verrouillé avec l'autre partie de contenant (2, 5), et une position de libération dans laquelle l'élément de verrouillage (8) est déverrouillé de l'autre partie de contenant (2, 5).

4. Fermeture pour un contenant médical stérile (1) selon l'une quelconque des revendications précédentes, **caractérisée en ce que** le volet de fermeture (6) présente un évidement (11) et l'élément de verrouillage (8) présente un évidement (38), dans laquelle, l'élément de verrouillage (8) se trouvant dans la position de verrouillage, un sceau (12) peut être aménagé pour sceller le contenant médical stérile (1) dans les évidements (11, 38).

5. Fermeture pour un contenant médical stérile (1) selon la revendication 4, **caractérisée en ce que** le sceau (12) est inséré à l'état monté dans l'évidement (38) de l'élément de verrouillage (8) et est affleurant à celui-ci.

6. Fermeture pour un contenant médical stérile (1) selon l'une quelconque des revendications précédentes, **caractérisée en ce que** la patte de fermeture (7) dans la position de fermeture de la fermeture est adjacente au volet de fermeture (6) et dans une position d'ouverture de la fermeture, est écartée du volet de fermeture (6).

7. Fermeture pour un contenant médical stérile (1) selon l'une quelconque des revendications précédentes, **caractérisée en ce que** la patte de fermeture (7) dans la position de fermeture de la fermeture, est entourée au moins partiellement par l'élément de verrouillage (8).

8. Fermeture pour un contenant médical stérile (1) selon l'une quelconque des revendications précédentes, **caractérisée en ce que** l'élément de verrouillage (8) est précontraint dans la position de verrouillage, en particulier au moyen d'un ressort (17).

9. Contenant médical stérile (1) comportant une fermeture selon l'une quelconque des revendications précédentes.
